# EUROPEAN PATENT APPLICATION

(11) **EP 2 508 187 A1**
(43) Date of publication of application: **10.10.2012**
(21) Application number: 12380016.1
(22) Date of filing: 04.04.2012
(51) Int. Cl.: A61K 31/616, A61K 47/40, A61K 9/14

(54) **Triflusal powder containing cyclodextrin as a stabilizing agent**

(30) Priority: 05.04.2011 ES 201100397
(71) Applicant: Simbec Iberica, SL, 08013 Barcelona (ES)
(72) Inventor: Sune Negre, José Maria, 08028 Barcelona (Barcelona) (ES); Mestre Castell, José, 08960 Sant Just Desvern (Barcelona) (ES)
(74) Representative: Torner Lasalle, Elisabet

(57) **Abstract**

The present invention proposes a stable triflusal compound powder for oral administration solid pharmaceutical forms, which contains, as a stabilizing excipient, at least one cyclodextrin selected from: β-cyclodextrin, α-cyclodextrin, dimethyl-β-cyclodextrin, γ-cyclodextrin, 2-hydroxyethylcyclodextrin, and mixtures thereof. The mentioned triflusal compound powder is used for producing granules, tablets or sachets. The mentioned triflusal compound powder is prepared by dry preparation.

## Description

### Technical Field

The invention relates to a pharmaceutic form of triflusal in powder form stabilized with cyclodextrins for packaging in sachets or for preparing tablets.

The invention also relates to the use of said powder for producing granules, tablets or sachets and to a method for preparing the mentioned triflusal compound powder.

Lastly the invention also describes the use of one or more cyclodextrins to stabilize the mentioned triflusal compound powder.

### Background of the Invention

Triflusal is a platelet aggregation inhibiting drug which was discovered and developed in Spain since 1976, described in patent US 4,096,252. Its chemical name is 2-(acetyloxy)-4-(trifluoromethyl)-benzoic acid and it has the following structure:

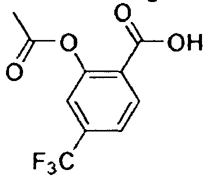

Triflusal acts by inhibiting prostanoid synthesis by means of acetylation causing the irreversible blockage of cyclooxygenase. This effect prevents the physiological synthesis of anti-aggregation factor (prostacyclin or PG12) and pro-aggregation factor (thromboxane A2 or TXA2), although the synthesis of anti-aggregation factor (prostacyclin) is less affected and recovers more quickly.

Indications authorized in Spain for preparations containing triflusal are: prophylaxis of coronary and cerebral thrombosis (myocardial infarction, myocardial reinfarction, cerebral thromboembolism, transitory ischemic attacks).

Triflusal is marketed today in the form of capsules containing 300 mg/capsule of said active ingredient, or in the form of powder contained in the stopper of the same packaging (vial), to dissolve it in the solvent of said vial.

Due to triflusal incompatibility with all the excipients commonly used in chemical and pharmaceutical technology, marketed capsules do not contain any excipient except for the gelatin of the capsule where triflusal powder is introduced and the ink printing thereon.

The formulation marketed as a powder for oral solution consists of single-dose packages containing the transparent solvent in the flask and the white triflusal powder inside the stopper such that the dose is reconstituted at the time of administration. This single-dose dosage form and reconstitution right before use is also bound by triflusal incompatibility with most of the excipients commonly used in chemical and pharmaceutical technology.

Therefore the two triflusal formulations on the market today have a series of administration problems due to the fact that formulating triflusal with an excipient of any type has not been achieved, mainly due to problems of incompatibility and instability.

### Brief description of the Invention

The problem to be solved with the present invention is that of providing an effective and safe alternative triflusal formulation which improves the formulations marketed today.

It has surprisingly been found that cyclodextrins stabilize triflusal being able to obtain a compound powder from same which is suitable to be packaged directly into sachets and also to be granulated and subsequently compressed for preparing tablets. This compound powder can also be used for dosing capsules, vials and for forming part of dosage form of any type suitable for that purpose.

In the present invention the triflusal is presented in the form of compound powder forming a mixture with cyclodextrins which surprisingly stabilize the triflusal preventing its degradation over time.

The presentation in the form of compound powder packaged in sachets, object of the present invention, is an original and unique presentation at present, since only triflusal preparations in the form of capsules and single-dose vials for reconstitution at the time of use are known on the market today.

The advantages of the triflusal compound powder object of the present invention can be summarized as the following:
a- greater stability since it has been demonstrated that the composite mixture with cyclodextrins surprisingly stabilizes triflusal over time.
b- simplifies preparation production since it is precisely a dry mixture, thereby avoiding kneading, wet granulation, sieving or granulating the compound powder once dried and an end mixture with extragranular products.

The objective of the invention therefore consists of obtaining triflusal formulation in the form of stable compound powder suitable for obtaining solid dosage forms for oral administration such that the triflusal is kept stable in said form until the expiration established for the preparation. Furthermore, a more comfortable triflusal administration is thereby provided, particularly in children and the elderly in whom administration by means of capsules represents an extreme difficulty in swallowing, more so if taking into account that the dose of 300 mg makes an accordingly large capsule size a must. The form presented in the invention is also more suitable than the powder-stopper/solvent vial form of the market, since the latter requires special attention to obtain complete dissolution of the triflusal-containing powder, it is also more expensive economically and is also less convenient for administration.

Therefore triflusal administration is simplified with the present invention, the preparation is more economical, easier to transport and administer, and is essentially stable as a result of using cyclodextrins which act as stabilizing excipients.

To achieve this objective it was necessary to solve the problem of triflusal stability which has been solved as a result of this invention, and with the studied technique and excipients, it has been presented in the most suitable pharmaceutic form (compound powder packaged in sachets) with the advantages described.

### Detailed Description of the Invention

Triflusal is known to be a very unstable drug, this instability further increasing when it is mixed with excipients for preparing different formulations.

In an attempt to solve the problem of triflusal instability with excipients, hard-to-apply technologies (micro- or nanoencapsulation, atomization, etc) were tested, but none of them gave any results in terms of stabilization.

It has surprisingly been found that triflusal was stable in combination with a single group of substances (cyclodextrin group) as demonstrated in the following tables.

Tables 2 and 3 show the results of the tested excipients which are dismissed due to poor stability or to characteristics (solubility) which are unsuitable for the dosage form. The stability of triflusal and excipient mixtures was determined by measuring the percentages of triflusal degradation product formed in the different tested time, temperature and moisture conditions by high-performance liquid chromatography (HPLC), following the conditions of the European Pharmacopeia monograph for triflusal. The degradation product which is quantified is the deacetylated product known as impurity B in European Pharmacopeia. Tests in which the percentage of degradation product exceeded 0.5% by weight were eliminated.

Table 4 shows the results of the excipients which have demonstrated to be valid due to their stability in the preparation formula and to their characteristics which are suitable for the dosage form presented, and which make up the object of the present invention. The commercial product DISGREN^{®} in the form of single-dose oral powder for reconstitution is used as a reference product for comparative purposes.

From the results observed, the design of 3 complete formulations suitable for administration in human beings, based on two excipients which have shown to stabilize triflusal over time depending on temperature, is set forth. These formulas are indicated in Table 5 and the experimental results of their degradation in Table 6.

The excipients used in all the stability tests were the following, which are indicated in Table 1:

**Table 1**

| | |
|---|---|
| Mannitol | Diluent; polyol |
| Glycine | pH regulator; amino acid |
| Anhydrous lactose | Diluent; disaccharide |
| Anhydrous sodium sulfate | Diluent, inorganic salt |
| Anhydrous sodium bicarbonate | Basic inorganic salt |
| Tribasic sodium citrate, hydrated A.I. | Basic pH regulator |
| Hydrated dibasic sodium citrate | pH regulator, dibasic salt |
| Anhydrous tribasic sodium phosphate | pH regulator, tribasic salt |
| Anhydrous dibasic sodium phosphate | pH regulator, dibasic salt |
| Monobasic sodium phosphate | pH regulator, monobasic salt |
| Arginine | Dibasic amino acid, basic pH |
| Anhydrous calcium carbonate | Inorganic salt |
| Anhydrous sodium carbonate | Inorganic salt |
| Anhydrous Aerosil | Adsorbent, glidant |
| Urea | basic pH |
| Weegum HV | Diluent, thickener |
| β-cyclodextrin | Diluent, adsorbent, complexing agent |
| α-cyclodextrin | Diluent, adsorbent, complexing agent |
| γ-cyclodextrin | Diluent, adsorbent, complexing agent |

**Table 2: Eliminated references/formulas. Experimental values of degradation products.**

| | T0 | T15 | T30 | T15 | T30 | |
|---|---|---|---|---|---|---|
| Reference/Formula | 25°C | 25°C/60% | 25°C/60% | 40°C/ 75% | 40°C/75% | Conclusion |
| TFS + Hydr. Dib. Na Citrate | 1.31% | Eliminated | - | - | - | |
| TFS + Hydr. Trib. Na Citrate (3717CQ) | 0.10% | 0.06% | 0.08% | 0.10% | 0.25% | Eliminated |
| TFS + Anhydrous Trib. Na Phosphate | 0.95% | Eliminated | - | - | - | |
| TFS + Anhydrous Trib. Na Phosphate + Anhydrous Dib. Na Phosphate | 1.24% | Eliminated | - | - | - | |
| TFS + Arginine | 0.17% | 0.35% | 0.48% | 8.52% | 4.64% | Eliminated |
| TFS + Anhydrous Na Carbonate | 0.28% | 0.74% | Eliminated | 2.47% | Eliminated | |
| TFS + Anhydrous Ca Carbonate | 0.18% | 0.60% | 0.81% | 2.09% | 2.17% | Eliminated |
| TFS + Anhydrous Dib.Na Phosphate | 2.24% | 3.95% | Eliminated | 2.22% | Eliminated | |
| TFS + Anhydrous Trib.Na Phosphate | 5.51 % | 12.12% | Eliminated | 0.42% | Eliminated | |
| TFS + Anhydrous Aerosil | 0.24% | 1.48% | Eliminated | 5.85% | Eliminated | |
| TFS + Urea | 0.06% | 0.21 % | 0.12% | 0.05% | 0.30% | Eliminated |
| TFS + Veegum HV | 2.68% | Eliminated | - | - | - | |
| TFS + Anhydrous Na | | | | | | |
| Bicarbonate | 7.05% | Eliminated | - | - | - | |

| | | | | | | |
|---|---|---|---|---|---|---|
| T0: time zero (start of stability study); T15, T30: 15 days and 30 days of stability study. 25°C/60%: stability at 25°C and at 60% humidity; 40°C/75%: stability at 40°C and at 75% humidity. | | | | | | |

The results of the table indicate the % by weight of triflusal degradation product formed in different tested conditions.

**Table 3: Dismissed references/formulas. Experimental values of degradation products.**

| Reference/Formula | T0 | T15 | T30 | T15 | T30 | Conclusion |
|---|---|---|---|---|---|---|
| | 25 °C | 25°C/60% | 25°C/60% | 40°C/75% | 40°C/75% | |
| Triflusal (blank) | 0.03 | 0.02 | 0.08 | 0.05 | 0.06 | |
| Triflusal + Mannitol | 0.06 | 0.07 | 0.24 | 0.05 | 0.13 | Eliminated |
| Triflusal + Glycine | 0.08 | 0.04 | 0.21 | 0.06 | 0.23 | Eliminated |
| Triflusal + Anhydrous lactose | 0.04 | 0.03 | 0.24 | 0.05 | 0.41 | Eliminated |
| Triflusal + Anhydrous sodium sulfate | 0.04 | 0.09 | 0.09 | 0.06 | 0.38 | Eliminated |
| Triflusal + glycine + mannitol | 0.04 | - | 0.06 | - | 2.52 | Eliminated |

| | | | | | | |
|---|---|---|---|---|---|---|
| T0: time zero (start of stability study); T15, T30: 15 days and 30 days of stability study. 25°C/60%: stability at 25°C and at 60% humidity; 40°C/75%: stability at 40°C and at 75% humidity. | | | | | | |

The results of the table indicate the % by weight of triflusal degradation product formed in different tested conditions.

**Table 4: Valid references/formulas. Experimental values of degradation products.**

| Reference/Formula | T0 | T15 | T30 | T15 | T30 |
|---|---|---|---|---|---|
| | 25 °C | 25°C/60% | 25°C/60% | 40°C/75% | 40°C/75% |
| Disgren oral powder | 0.11 | 0.12 | 0.26 | 0.23 | 0.20 |
| Triflusal + hydrated tribasic sodium citrate | 0.10 | 0.06 | 0.08 | 0.10 | 0.25 |
| Triflusal + β-cyclodextrin (1/1 by weight) | 0.10 | 0.04 | 0.08 | 0.06 | 0.11 |
| Triflusal + β-cyclodextrin (1/4 by weight) | 0.08 | 0.08 | 0.10 | 0.09 | 0.09 |
| Triflusal + hydrated tribasic sodium citrate. Analytic purity QC3801 | 0.08 | - | 0.09 | - | 0.49 |
| Triflusal + β-cyclodextrin + hydrated sodium citrate A.I. | 0.04 | - | 0.09 | - | 0.32 |
| Triflusal + α-cyclodextrin | 0.12 | 0.12 | 0.12 | 0.13 | 0.13 |
| Triflusal + γ-cyclodextrin | 0.13 | 0.13 | 0.14 | 0.13 | 0.13 |

| | | | | | |
|---|---|---|---|---|---|
| T0: time zero (start of stability study); T15, T30: 15 days and 30 days of stability study. 25°C/60%: stability at 25°C and at 60% humidity; 40°C/75%: stability at 40°C and at 75% humidity. | | | | | |

The results of the table indicate the % by weight of triflusal degradation product formed in different tested conditions.

**Table 5**

| Formula F20 | Formula F21 | Formula F22 |
|---|---|---|
| 600 mg triflusal | 600 mg triflusal | 600 mg triflusal |
| 600 mg tribasic citrate | 600 mg tribasic citrate | 600 mg β-cyclodextrin |
| 30 mg saccharine | 600 mg β-cyclodextrin | 30 mg saccharine |
| 50 mg orange flavoring | 30 mg saccharine | 50 mg orange flavoring |
| 4 mg glycyrrhetinate | 50 mg orange flavoring | 4 mg glycyrrhetinate |
| | 4 mg glycyrrhetinate | |

**Table 6**

| | 25 °C /60 % HR | | | | 40 °C /75 % HR | | |
|---|---|---|---|---|---|---|---|
| | T = 0 | T = 1m | T = 3m | T = 6m | T = 1m | T = 3m | T = 6m |
| F20 | 0.12 | 6 | - | - | 98.4 | - | - |
| F21 | 0.07 | 0.15 | 0.22 | 0.40 | 1.57 | 100 | 100 |
| F22 | 0.07 | 0.08 | 0.1 | 0.19 | 0.2 | 0.83 | 3.09 |

| | | | | | | | |
|---|---|---|---|---|---|---|---|
| T = 0: time zero (start of stability study); T = 1m (1 month); T = 3 m (3 months); T = 6 m (6 months) of stability study. 25°C/60%: stability at 25°C and at 60% humidity; 40°C/75%: stability at 40°C and at 75% humidity. | | | | | | | |

The results of the table indicate the % by weight of triflusal degradation product formed in different tested conditions.

After conducting the studies and tests, it has been found that the triflusal powder-stabilizing excipients are those of the family of cyclodextrins: β-cyclodextrin, α-cyclodextrin, dimethyl-β-cyclodextrin, γ-cyclodextrin or 2-hydroxyethylcyclodextrin.

Particularly, the excipient β-cyclodextrin is the most compatible with triflusal, being the most effective within the group of cyclodextrins in terms of stabilizing triflusal over time and in function of the temperature. It is surprising to find a diluent excipient capable of maintaining correct triflusal stability in solid form, which was unknown in the state of the art.

The ratio by weight of triflusal and cyclodextrin in the powder of the formulation is comprised between 1/1 and 1/5.

The triflusal powder formulation stabilized with cyclodextrins is further flavored and sweetened with orange flavoring and sodium saccharine, respectively. The method for preparing the triflusal compound powder is by dry preparation, preferably in a room conditioned to that end. This stabilized triflusal powder is packaged directly into sachets with doses of triflusal comprised between 300 and 800 mg and a total weight per sachet comprised between 1.0 g and 5.0 g.

The preferred triflusal doses for the powder formulations of the present invention are comprised between 250 and 800 mg.

Consequently the following formula per sachet is preferred:

**Table 7**

| | |
|---|---|
| Triflusal | 600 mg |
| β-cyclodextrin | 600 mg |
| Sodium saccharine | 30 mg |
| Orange flavoring | 50 mg |
| Glycyrrhetinate | 4 mg |

The production method used is the following (in an air-conditioned room):
1. Sieve β-cyclodextrin and triflusal through a 600 µm sieve.
2. Stir the two components above at approximately 2500 rpm for 10 minutes with a rod stirrer.
3. Sieve the rest of components (saccharine, orange flavoring and glycyrrhetinate) through a 600 µm sieve. Before sieving the saccharine, spray in a mortar.
4. Put all the components in a packaging and mix in a bi-conical drum for 15 minutes.
5. Dose in Alu-Alu sachets and seal.

Stabilized triflusal powder can also be granulated and the compressed granule gives rise to stable triflusal tablets.

### Examples

### Example 1

Formula per sachet, contemplating the preferred formula

**Table 8**

| | |
|---|---|
| Triflusal | 600 mg |
| Sodium saccharine | 30 mg |
| Orange flavoring | 50 mg |
| β-cyclodextrin | q.s. 1250 to 1650 mg |

The production technique followed is the same as that used in the preferred formula.

### Example 2

Formula per sachet:

**Table 9**

| | |
|---|---|
| Triflusal | 600 mg |
| Sodium saccharine | 30 mg |
| Orange flavoring | 50 mg |
| β-cyclodextrin | 600 to 1000 mg |

The production technique followed is the same as that used in the preferred formula.

### Example 3

Formula per sachet:

**Table 10**

| | |
|---|---|
| Triflusal | 600 mg |
| Sodium saccharine | 30 mg |
| Orange flavoring | 50 mg |
| Glycyrrhetinate | 4 mg |
| β-cyclodextrin | 300 to 800 mg |

The production technique followed is the same as that used in the preferred formula.

### Example 4

Formula per tablet:

**Table 11**

| | |
|---|---|
| Triflusal | 300 mg |
| β-cyclodextrin | 300 mg |
| Microcrystalline cellulose | 300 mg |
| Croscarmellose sodium | 50 mg |
| Talc | 40 mg |
| Anhydrous colloidal silica | 10 mg |

The production method used is the following:
1. Sieve β-cyclodextrin and triflusal through a 600 µm sieve.
2. Stir the two components above at approximately 2500 rpm for 10 minutes with a rod stirrer.
3. Sieve the rest of the components through a 600 µm sieve.
4. Mix in a bi-conical drum or a suitable mixer for 15 minutes.
5. Compress with oblong punches and to a unit weight of 1000 mg/tablet.

## Claims

1. A triflusal compound powder in a stable form, for oral administration solid pharmaceutic forms, **characterized in that** it contains at least one cyclodextrin as a stabilizing excipient.

2. The triflusal compound powder according to claim 1, **characterized in that** the stabilizing cyclodextrin is selected from: β-cyclodextrin, α-cyclodextrin, dimethyl-β-cyclodextrin, γ-cyclodextrin, 2-hydroxyethylcyclodextrin, and mixtures thereof.

3. The triflusal compound powder according to claim 1 or 2, **characterized in that** the stabilizing cyclodextrin is a β-cyclodextrin.

4. The triflusal compound powder according to any claim 1 to 3, **characterized in that** the ratio by weight of triflusal to cyclodextrin is comprised between 1/1 and 1/5.

5. The triflusal compound powder according to any claim 1 to 4, **characterized in that** it comprises other excipients and/or aromas and/or sweeteners.

6. Use of a triflusal compound powder according to amy claim 1 to 5 for producing granules, tablets or sachets.

7. A granule, tablet or sachet obtained with a triflusal compound powder according to any claim 1 to 5.

8. A granule, tablet or sachet obtained from a triflusal compound powder according to claim 7, **characterized in that** the dose of triflusal is comprised between 300 and 800 mg.

9. A sachet according to claim 6 or 7, **characterized in that** the powder is packaged in sachets with a total weight per sachet comprised between 1.0 g and 5.0 g.

10. A method for preparing a triflusal compound powder according to any claim 1 to 5, **characterized in that** the preparation is carried out by dry preparation.

11. The method according to claim 10, **characterized in that** the powder is granulated by dry granulation.

12. A tablet obtained by compressing a granule of triflusal compound powder according to claim 11, **characterized in that** it contains triflusal at doses comprised between 250 and 800 mg.

13. The tablet obtained by compressing a granule of triflusal compound powder according to claim 12, **characterized in that** it further contains citrates as pH regulators.

14. Use of one or more cyclodextrins to stabilize triflusal in a pharmaceutical formulation.

15. Use according to claim 14, **characterized in that** the cyclodextrin is selected from: β-cyclodextrin, α-cyclodextrin, dimethyl-β-cyclodextrin, γ-cyclodextrin, 2-hydroxyethylcyclodextrin, and mixtures thereof.
